# EUROPÄISCHE PATENTSCHRIFT

(11) **EP 1 052 945 B1**
(45) Veröffentlichungstag und Bekanntmachung des Hinweises auf die Patenterteilung: **02.08.2006**
(21) Anmeldenummer: 99973250.6
(22) Anmeldetag: 03.11.1999
(51) Int. Cl.: A61B 17/28

(54) **MEDIZINISCHES ROHRSCHAFTINSTRUMENT**
TUBULAR MEDICAL INSTRUMENT
INSTRUMENT MEDICAL A TIGE TUBULAIRE

(30) Priorität: 04.12.1998 DE 19855968
(43) Veröffentlichungstag der Anmeldung: 22.11.2000
(73) Patentinhaber: Karl Storz GmbH & Co. KG, 78532 Tuttlingen (DE)
(72) Erfinder: BACHER, Uwe, D-78532 Tuttlingen (DE); ANDERS, Fridolin, D-78194 Immendingen (DE)
(74) Vertreter: Heuckeroth, Volker
(86) Internationale Anmeldenummer: PCT/EP1999/008399
(87) Internationale Veröffentlichungsnummer: WO 2000/033749

(56) Entgegenhaltungen:
- EP-A- 0 688 535
- WO-A-98/37818
- DE-U- 7 330 291
- DE-U- 7 332 292
- DE-U- 29 512 503
- US-A- 4 982 727

## Beschreibung

Die Erfindung betrifft ein medizinisches Rohrschaftinstrument, mit einem langerstreckten Rohrschaft, mit zumindest einem beweglichen Werkzeug am distalen Ende, weiterhin mit zumindest einem beweglichen Griffteil am proximalen Ende, und schließlich mit einem langerstreckten Kraftübertragungselement, dessen distales Ende mit dem zumindest einen Werkzeug und dessen proximales Ende mit dem zumindest einen beweglichen Griffteil kraftschlüssig verbunden ist, wobei sich das Kraftübertragungselement durch den Rohrschaft erstreckt.

Ein derartiges medizinisches Rohrschaftinstrument ist allgemein bekannt.

Derartige Rohrschaftinstrumente werden in der minimal-invasiven Chirurgie als Operationsinstrumente eingesetzt, um operative Eingriffe durch eine kleine Körperinzision unter endoskopischer Kontrolle durchzuführen. Für unterschiedliche durchzuführende operative Eingriffe im menschlichen oder tierischen Körper werden solche Rohrschaftinstrumente mit unterschiedlichen Funktionen ausgestattet.

Unter einem Rohrschaftinstrument im Sinne der vorliegenden Erfindung wird demnach bspw. eine Zange zum Schneiden und/oder Fassen und/oder bspw. auch ein Nadelhalter verstanden. Das zumindest eine beweglichen Werkzeug kann demnach bspw. in Form eines beweglichen Maulteils ausgebildet sein, das eine Schneide aufweist, die mit einer Schneide eines zweiten beweglichen oder unbeweglichen Maulteils schneidend zusammenwirkt. Das zumindest eine bewegliche Werkzeug kann auch ein Maulteil sein, das eine stumpfe Fläche aufweist, die mit einem zweiten Maulteil zum Fassen von Gewebe zusammenwirkt. Im Falle eines Nadelhalters weist das zumindest eine bewegliche Werkzeug eine Ausgestaltung auf, die es ermöglicht, mit diesem Werkzeug eine Nadel im Operationsgebiet zu halten und zur Bildung einer Naht zum Verbinden von Gewebe zu führen.

Zur Betätigung des zumindest einen beweglichen Werkzeuges ist am proximalen Ende des Rohrschaftinstrumentes zumindest ein bewegliches Griffteil vorgesehen, das mit dem zumindest einen beweglichen Werkzeug über ein langerstrecktes Kraftübertragungselement, bspw. in Form einer Zugstange, kraftschlüssig verbunden ist. Eine Bewegung des beweglichen Griffteils bewirkt dann eine Bewegung des zumindest einen beweglichen Werkzeugs am distalen Ende des Rohrschafts, um die entsprechende Funktion, bspw. Schneiden oder Halten und Führen der Nadel durch das Gewebe, zu bewirken.

Im Sinne der minimal-invasiven Chirurgie ist es mitunter erforderlich, solche Rohrschaftinstrumente mit einem Rohrschaftdurchmesser auszubilden, der etwa 3 mm oder weniger beträgt. Solch kleine Rohrschaftdurchmesser sind insbesondere bei der Operation an Kleinkindern oder auch bei Eingriffen in bestimmten Operationsbereichen, wie im Kopfbereich, geboten.

Bei den bekannten Rohrschaftinstrumenten, insbesondere solchen mit einem Durchmesser von etwa 3 mm und weniger, ist das Kraftübertragungselement im Durchmesser an den Innendurchmesser des Rohrschaftes angepaßt, d.h. der Außendurchmesser des Kraftübertragungselementes ist etwa so groß wie der Innendurchmesser des Rohrschaftes, so daß das Kraftübertragungselement den Innenraum des Rohrschaftes vollständig ausfüllt.

Der Nachteil dieser Ausgestaltung besteht darin, daß der Innenraum des Rohrschaftes bei eingebautem Kraftübertragungselement nicht ausreichend gespült werden kann, da zwischen dem Kraftübertragungselement und dem Rohrschaft kein ausreichender Durchgang für eine Spülflüssigkeit vorhanden ist. Da solche miniaturisierten Rohrschaftinstrumente jedoch nicht in der Weise zerlegbar, zumindest nicht so leicht zerlegbar sind, daß das Kraftübertragungselement zum Reinigen aus dem Rohrschaft entnommen werden kann, weil die Verbindung zwischen dem zumindest einen beweglichen Werkzeug und dem Kraftübertragungselement einerseits und dem Kraftübertragungselement und dem zumindest einen beweglichen Griffteil andererseits nicht oder nicht leicht lösbar ist, bedeutet dies, daß die bekannten Rohrschaftinstrumente nur ungenügend reinigbar sind. Sich während einer Operation im Innenraum des Rohrschaftes ansammelnde Verschmutzungen können daher nicht soweit entfernt werden, daß diese Rohrschaftinstrumente den strengen Sterilitäts- und Hygieneanforderungen entsprechen können.

Es wurde zur Verbesserung der Reinigungseigenschaften dieser Rohrschaftinstrumente in Betracht gezogen, das Kraftübertragungselement mit einem Außendurchmesser auszugestalten, der geringer ist als der Innendurchmesser des Rohrschaftes, so daß ein genügender Spülraum zwischen dem Kraftübertragungselement und dem Rohrschaft verbleibt. Dabei hat es sich jedoch als besonders nachteilig herausgestellt, daß sich der Rohrschaft bei hohen, von dem Kraftübertragungselement auf das zumindest eine bewegliche Werkzeug übertragenen Zugkräften trotz seiner starren Ausgestaltung verbiegt. Das Rohrschaftinstrument wird jedoch unbrauchbar, wenn der Rohrschaft verbogen ist. Der gleiche nachteilige Effekt stellte sich auch dann ein, wenn anstelle eines im Querschnitt runden Kraftübertragungselementes kleinen Durchmessers ein beidseitig abgeflachtes Zugband eingesetzt wurde. Auch ein Kraftübertragungselement in Form eines flachen Zugbandes führte zu einem Stabilitätsverlust des Rohrschaftes.

Aus dem dentschen Gebrauchmuster DE-4-7330291 ist ein Operations instrument bekannt, bei dem Abstandskugeln oder - rollen in gewissen Abständen auf dem Schaft aufgesetzt sind, um ihn im Kohlschaft zu fürhen.

Der Erfindung liegt daher die Aufgabe zugrunde, ein Rohrschaftinstrument der eingangs genannten Art dahingehend weiterzubilden, daß das Rohrschaftinstrument durch Spülen des Innenraums des Rohrschaftes bei eingebautem Kraftübertragungselement leicht reinigbar ist, und daß die Stabilität des Rohrschaftinstrumentes gegen ein Verbiegen des Rohrschafts weiterhin gewährleistet ist.

Erfindungsgemäß wird diese Aufgabe hinsichtlich des eingangs genannten Rohrschaftinstrumentes dadurch gelöst, daß das Kraftübertragungselement zumindest in seinem sich durch den Rohrschaft erstreckenden Bereich zumindest abschnittsweise an zumindest drei Umfangsstellen radial bis an eine Innenwand des Rohrschaftes reicht und zwischen den drei Umfangsstellen von der Innenwand einen lichten Abstand aufweist, und in seinen übrigen, sich durch den Rohrschaft erstreckenden Abschnitten zumindest teilumfänglich einen lichten Abstand von der Innenwand aufweist.

Erfindungsgemäß ist demnach zunächst vorgesehen, daß das Kraftübertragungselement zumindest in seinem sich durch den Rohrschaft erstreckenden Bereich durchgehend zumindest teilumfänglich einen lichten Abstand von der Innenwand des Rohrschaftes aufweist, so daß der Innenraum des Rohrschaftes auch bei eingebautem Kraftübertragungselement einen zur Reinigung ausreichenden Spülquerschnitt aufweist. Weiterhin ist erfindungsgemäß vorgesehen, daß das Kraftübertragungselement axial zumindest abschnittsweise an zumindest drei Umfangsstellen radial bis an die Innenwand des Rohrschaftes reicht. Insbesondere bei Rohrschaftinstrumenten mit einem Rohrschaftdurchmesser von etwa 3 mm und weniger hat sich nämlich die Erkenntnis eingestellt, daß bei solch dünnen Rohrschäften dem Kraftübertragungselement auch die Aufgabe zukommt, den Rohrschaft gegen ein Verbiegen bei der Übertragung hoher Zugkräfte zu stabilisieren. Dies wird bei dem erfindungsgemäßen Rohrschaftinstrument durch die Ausgestaltung des Kraftübertragungselementes dadurch gewährleistet, daß dieses zumindest abschnittsweise an drei Umfangsstellen radial bis an die Innenwand des Rohrschaftes reicht und den Rohrschaft dadurch stabilisiert. Um auch in diesen Abschnitten einen Durchgang für eine Spülflüssigkeit zu gewährleisten, weist das Kraftübertragungselement in diesen Abschnitten zwischen den Umfangsstellen zur Innenwand des Rohrschaftes einen lichten Abstand für den Durchtritt einer Spülflüssigkeit auf. Während es sich bei den im Stand der Technik in Betracht gezogenen Lösungen, das Kraftübertragungselement mit einem geringeren Durchmesser oder als flaches Band auszugestalten, herausgestellt hat, daß ein solches Kraftübertragungselement bei der Betätigung des Rohrschaftinstrumentes in dem Rohrschaft hin- und herspringt und somit ein Verbiegen des Rohrschaftes verursacht, wird ein solches Hin- und Herspringen bei dem erfindungsgemäßen Kraftübertragungselement durch die radial bis an dei Innenwand des Rohrschaftes reichenden Umfangsstellen vermieden. Gleichzeitig ist das Kraftübertragungselement des erfindungsgemäßen Rohrschaftinstrumentes selbst stabiler ausgebildet und kann daher höhere Zugkräfte übertragen als ein Zugband oder ein dünner Draht. Im einfachsten Fall kann das Kraftübertragungselement gemäß der vorliegenden Erfindung in den axialen Abschnitten, an denen das Kraftübertragungselement an die Innenwand des Rohrschaftes reicht, im Querschnitt etwa dreieckig ausgebildet sein. Das erfindungsgemäße Rohrschaftinstrument ist aufgrund des zwischen dem Kraftübertragungselement und dem Rohrschaft vorhandenen durchgehenden Spülkanal leicht reinigbar, und trotz des bestehenden durchgehenden Spülraumes tritt kein Stabilitätsverlust des Rohrschaftes auf.

Somit wird die der Erfindung zugrundeliegende Aufgabe vollkommen gelöst.

In einer bevorzugten Ausgestaltung erstrecken sich die zumindest drei radial bis an die Innenwand des Rohrschaftes reichenden Umfangsstellen durchgehend über den gesamten, sich durch den Rohrschaft erstreckenden Bereich des Kraftübertragungselementes.

Bei dieser Ausgestaltung erstrecken sich die zumindest drei Umfangsstellen nicht nur abschnittsweise über das Kraftübertragungselement, sondern über den gesamten sich in dem Rohrschaft erstreckenden Bereich des Kraftübertragungselementes. Hierdurch wird der Vorteil erreicht, daß der Rohrschaft über seine gesamte Länge eine Stabilisierung durch das Kraftübertragungselement erfährt. Ein weiterer Vorteil besteht darin, daß die Herstellung des Kraftübertragungselementes vereinfacht wird. Beispielsweise kann das Kraftübertragungselement aus einem zylindrischen Vollmaterial durch Einbringen von zumindest drei durchgehenden Abflachungen oder Vertiefungen in axialer Richtung in einem materialabtragenden Verfahren, bspw. durch Lasertechniken oder Funkenerosion, oder in einem nicht materialabtragenden Verfahren, bspw. durch Rollen oder Profilziehen, d.h. Formen durch Ziehen eines Rundmaterials durch eine entsprechende Matrize, hergestellt werden.

In einer weiteren bevorzugten Ausgestaltung ist das Kraftübertragungselement an den zumindest drei Umfangsstellen abgerundet.

Hierbei ist von Vorteil, daß die zumindest drei bis an die Innenwand des Rohrschaftes reichenden Umfangsstellen des Kraftübertragungselementes im Langzeitgebrauch des Rohrschaftinstrumentes nicht in die Innenwand des Rohrschafts einschneiden, wie dies bei scharfen Kanten möglicherweise der Fall sein könnte.

Dabei ist es bevorzugt, wenn der Krümmungsradius der Umfangsstellen kleiner ist als der Innenradius des Rohrschafts.

Hierbei ist von Vorteil, daß das Kraftübertragungselement mit seinen zumindest drei Umfangsstellen nur linienförmig an der Innenwand des Rohrschafts anliegt, wodurch die Reibung bei der axial hin- und hergehenden Bewegung des Kraftübertragungselementes in dem Rohrschaft beim Gebrauch des Rohrschaftinstrumentes minimiert wird. Außerdem wird der für den Durchgang einer Spülflüssigkeit vorhandene Spülquerschnitt zwischen dem Kraftübertragungselement und dem Rohrschaft vergrößert, und es werden für die Spülflüssigkeit schwer zugängliche Bereiche zwischen der Außenkontur des Kraftübertragungselementes und der Innenwand des Rohrschaftes minimiert.

In einer weiteren bevorzugten Ausgestaltung ist das Kraftübertragungselement zwischen den zumindest drei Umfangsstellen zur Längsmittelachse des Kraftübertragungselementes hin gesehen konkav vertieft.

Hierbei ist von Vorteil, daß der für den Durchgang einer Spülflüssigkeit nutzbare Spülquerschnitt gegenüber einer Ausgestaltung, bei der der Querschnitt des Kraftübertragungselementes die Form eines Dreiecks mit geradlinigen Schenkeln aufweist, ohne Stabilitätsverlust noch weiter vergrößert werden kann, so daß die Reinigbarkeit des erfindungsgemäßen Rohrschaftinstrumentes weiter verbessert sind.

In einer weiteren bevorzugten Ausgestaltung erstrecken sich die zumindest drei radial bis an die Innenwand des Rohrschaftes reichenden Umfangsstellen geradlinig.

Hierbei ist von Vorteil, daß sich das Kraftübertragungselement einfach herstellen läßt, wobei jedoch auch in Betracht gezogen werden kann, daß die zumindest drei genannte Umfangsstellen schraubenlinienförmig um die Längsmittelachse des Kraftübertragungselementes verlaufen.

In einer weiteren bevorzugten Ausgestaltung nehmen die zumindest drei Umfangsstellen einen gleichen Winkelabstand zueinander ein.

Durch diese Ausgestaltung wird eine optimale allseitige und symmetrische Abstützung des Kraftübertragungselementes an der Innenwand des Rohrschaftes erzielt.

Die erfindungsgemäße Ausgestaltung des Rohrschaftinstrumentes läßt sich bei einer Zange zum Schneiden und/oder Fassen ebenso nutzen wie bei einem Nadelhalter.

Weitere Vorteile ergeben sich aus der nachfolgenden Beschreibung und der beigefügten Zeichnung.

Ausführungsbeispiele der Erfindung sind in der Zeichnung dargestellt und werden in der nachfolgenden Beschreibung näher erläutert. Es zeigen:
- Fig. 1: eine Seitenansicht eines medizinischen Rohrschaftinstrumentes gemäß einem ersten Ausführungsbeispiel;
- Fig. 2: einen Querschnitt entlang der Linie II-II in Fig. 1 in vergrößertem Maßstab;
- Fig. 3: einen Fig. 2 entsprechenden Querschnitt, der den Stand der Technik zeigt;
- Fig. 4: einen Schnitt entlang der Linie IV-IV in Fig. 2 in einem in Fig. 1 mit A bezeichneten Ausschnitt des Rohrschaftinstrumentes in Fig. 1; und
- Fig. 5: eine Seitenansicht auf ein Rohrschaftinstrument gemäß einem weiteren Ausführungsbeispiel.

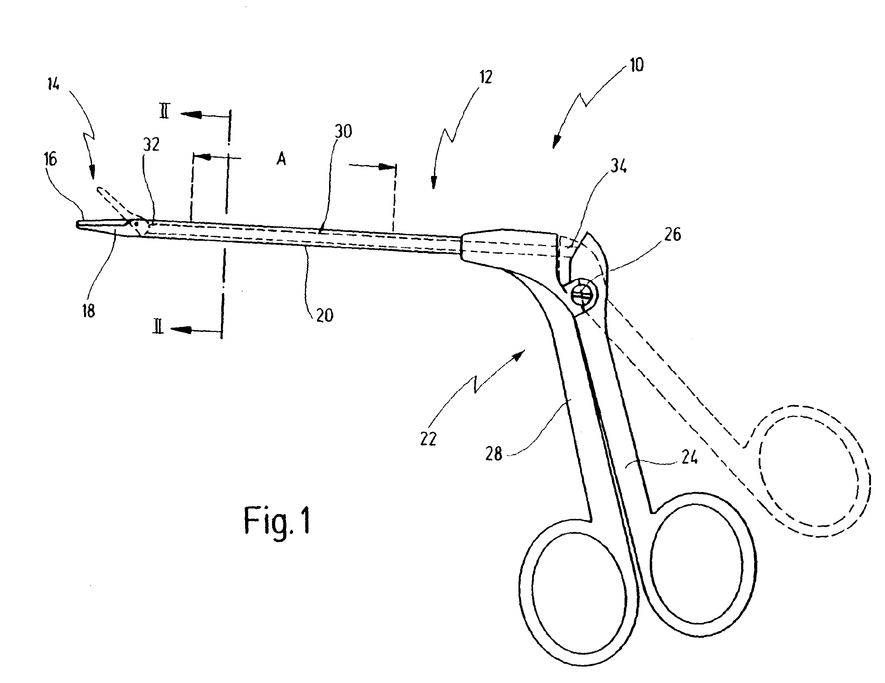

In Fig. 1 ist ein mit dem allgemeinen Bezugszeichen 10 versehenes medizinisches Rohrschaftinstrument dargestellt. Das medizinische Rohrschaftinstrument 10 ist gemäß dem gezeigten Ausführungsbeispiel eine medizinische Zange 12 zum Schneiden von Gewebe im menschlichen oder tierischen Körper.

Die Zange 12 weist am distalen Ende zumindest ein bewegliches Werkzeug 14 auf. Das Werkzeug 14 ist ein bewegliches Maulteil 16, das mit einem weiteren Maulteil 18, das in diesem Fall unbeweglich ist, aber auch beweglich sein kann, schneidend zusammenwirkt.

Die beiden Maulteile 16 und 18 sind am distalen Ende eines Rohrschaftes 20 befestigt. Der Rohrschaft 20 ist hohlzylindrisch ausgestaltet. Der Rohrschaft 20 weist im vorliegenden Fall ferner einen Außendurchmesser von etwa 3 mm oder weniger auf. Der Rohrschaft 20 ist weiterhin starr.

Am proximalen Ende des Rohrschafts 20 ist mit diesem eine Handhabe 22 fest verbunden. Die Handhabe 22 weist zumindest ein bewegliches Griffteil 24 auf, das der Betätigung des zumindest einen beweglichen Werkzeugs 14, d.h. hier des einen Maulteils 16, dient.

Das bewegliche Griffteil 24 ist über ein Gelenk 26 gelenkig mit einem unbeweglichen, mit dem Rohrschaft 20 fest verbundenen Griffteil 28 verbunden.

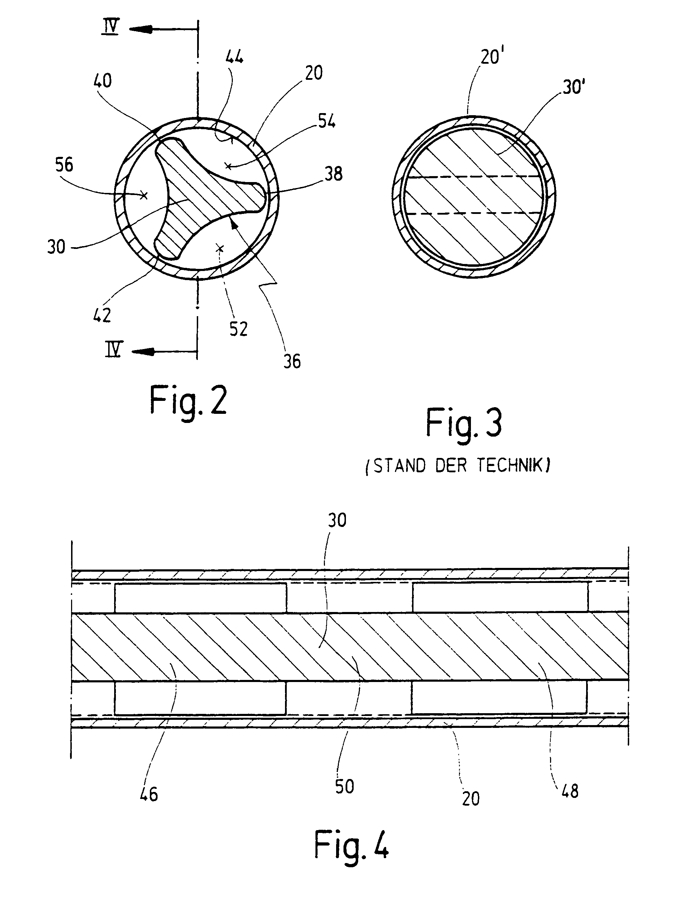

Zur Kraftübertragung von dem beweglichen Griffteil 24 auf das bewegliche Maulteil 16 ist ein Kraftübertragungselement 30 vorgesehen, das in Fig. 1 durch unterbrochene Linien angedeutet ist und in Fig. 2 und 4 näher dargestellt ist.

Das Kraftübertragungselement 30 ist zwischen das zumindest eine bewegliche Werkzeug 14, d.h. hier das bewegliche Maulteil 16 und das zumindest eine bewegliche Griffteil 24 geschaltet. Genauer gesagt ist ein distales Ende 32 des Kraftübertragungselementes 30 mit dem beweglichen Maulteil 16 und ein proximales Ende 34 des Kraftübertragungselementes 30 mit dem beweglichen Griffteil 24 verbunden. Das Kraftübertragungselement 30 erstreckt sich dabei durch den Rohrschaft 20, d.h. ist in dem Rohrschaft 20 angeordnet.

Das Kraftübertragungselement 30 arbeitet zum Schließen der Maulteile 16 und 18 auf Zug, d.h. aus der in Fig. 1 mit unterbrochenen Linien dargestellten Offenlage des beweglichen Maulteiles 16, der die mit unterbrochenen Linien dargestellte Stellung des beweglichen Griffteils 24 entspricht, wird durch Bewegen des beweglichen Griffteils 24 auf das unbewegliche Griffteil 28 zu das Kraftübertragungselement 30 in dem Rohrschaft 20 nach proximal gezogen, wodurch das bewegliche Maulteil 16 gegen das unbewegliche Maulteil 18 geschlossen wird.

Wie aus Fig. 2 und 4 nun hervorgeht, die das Kraftübertragungselement im Querschnitt und im Längsschnitt in vergrößertem Maßstab darstellen, weist das Kraftübertragungselement 30 zumindest in seinem sich durch den Rohrschaft 20 erstreckenden Bereich eine Außenkontur 36 auf, die axial zumindest abschnittsweise an zumindest drei Umfangsstellen, die in Fig. 2 mit 38, 40 und 42 bezeichnet sind, radial bis an eine Innenwand 44 des Rohrschafts 20 reicht und dadurch eine allseitige Abstützung des Rohrschaftes 20 gewährleistet.

Gemäß Fig. 4 sind die zumindest drei Umfangsstellen 38, 40 und 42 an zwei axialen Abschnitten 46 und 48 des Kraftübertragungselementes 30 vorgesehen. Da Fig. 4 jedoch nur einen axialen Ausschnitt des Rohrschaftes 20 und des Kraftübertragungselementes 30 darstellt, versteht es sich, daß derartige Abschnitte in größerer Anzahl zumindest in dem sich durch den Rohrschaft 20 erstreckenden Bereich des Kraftübertragungselementes 30 vorgesehen sind.

In den Abschnitten 46 und 48 erstrecken sich die radial an die Innenwand 44 des Rohrschaftes 20 reichenden Umfangsstellen 38, 40 und 42 über eine axiale Teillänge des Kraftübertragungselementes 30. In den übrigen Abschnitten, in denen das Kraftübertragungselement 30 keine bis an die Innenwand 44 des Rohrschaftes 20 reichende und sich an dieser abstützende Umfangsstellen aufweist, weist das Kraftübertragungselement 30 zumindest teilumfänglich einen lichten Abstand von der Innenwand 44 auf, wie in Fig. 4 mit einem Abschnitt 50 beispielhaft dargestellt ist, in dem das Kraftübertragungselement 30 allseitig einen lichten Abstand von der Innenwand 44 des Rohrschaftes 20 aufweist. Während das Kraftübertragungselement 30 in den Abschnitten 46 und 48 und den weiteren diesen Abschnitten entsprechenden Abschnitten einen im wesentlichen dreieckigen Querschnitt aufweist, kann das Kraftübertragungselement 30 in dem Abschnitt 50 und den weiteren diesen Abschnitt 50 entsprechenden Abschnitten einen im wesentlichen runden Querschnitt aufweisen, wobei der runde Querschnitt in dem Abschnitt 50 und den weiteren diesem Abschnitt 50 entsprechenden Abschnitten einen geringeren Durchmesser aufweist als die Innenwand 44 des Rohrschaftes 20.

Anstatt die zumindest drei Umfangsstellen 38, 40 und 42 an dem Kraftübertragungselement 30 nur axial abschnittsweise vorzusehen, ist es bevorzugt, wenn sich die an der Innenwand 44 des Rohrschaftes 20 abstützenden Umfangsstellen 38, 40 und 42 durchgehend über den gesamten sich durch den Rohrschaft 20 erstreckenden Bereich des Kraftübertragungselementes 30 erstrekken. Dies ist in Fig. 4 mit unterbrochenen Linien dargestellt. Mit anderen Worten weist dann das Kraftübertragungselement 30 an jeder beliebigen axialen Position einen Querschnitt auf, wie er in Fig. 2 dargestellt ist.

Die in der Art von Stegen ausgebildeten Umfangsstellen 38, 40, 42 erstrecken sich dabei zwischen dem proximalen Ende 34 und dem distalen Ende 32 geradlinig. Es kann jedoch auch eine gewendelte Ausgestaltung vorgesehen sein.

Zwischen den Umfangsstellen 38, 40 und 42 weist die Außenkontur 36 des Kraftübertragungselementes 30 radial jeweils einen lichten Abstand von der Innenwand 44 des Rohrschaftes 20 auf. Zwischen den an der Innenwand 44 des Rohrschaftes 20 anliegenden Umfangsstellen 38, 40 und 42 ist demnach jeweils ein lichter Zwischenraum 52, 54, 56 vorhanden, der als Spülkanal für den Durchgang einer Spülflüssigkeit dient. Die als Spülkanäle nutzbaren Zwischenräume 52, 54, 56 erstrecken sich somit über die gesamte Länge des in dem Rohrschaft 20 angeordneten Bereich des Kraftübertragungselementes 30.

An den Umfangsstellen 38, 40, 42 ist das Kraftübertragungselement 30 abgerundet. Dabei ist der Krümmungsradius der Umfangsstellen 38, 40, 42 kleiner als der Innenradius des Rohrschaftes 20.

Weiterhin weist die Außenkontur 36 des Kraftübertragungselements 30 zwischen den Umfangsstellen 38, 40, 42 eine zur Längsmittelachse des Kraftübertragungselementes 30 hin gesehen konkave Vertiefung auf, wodurch der Spülquerschnitt in den Zwischenräumen 52, 54, 56 vergrößert ist.

Ferner weisen die Umfangsstellen 38, 40 und 42 zueinander jeweils einen Winkelabstand von etwa 120° auf, d.h. sie nehmen einen gleichen Winkelabstand zueinander ein, wodurch eine gleichmäßige allseitige Abstützung des Kraftübertragungselementes 30 an der Innenwand 44 des Rohrschafts 20 erreicht wird.

Das Kraftübertragungselement 30 ist insgesamt massiv aus einem Vollmaterial hergestellt, bspw. aus einem zylindrischen Körper, in dem in die Außenkontur gemäß der Ausgestaltung in Fig. 2 durch materialabtragende Formgebung, bspw. durch ein Laserverfahren oder ein Funkenerosionsverfahren, teilumfänglich Vertiefungen eingebracht werden. Alternativ dazu kann das Kraftübertragungselement 30 mit der dargestellten Formgebung auch in einem nicht materialabtragenden Verfahren durch Rollen oder auch durch Profilziehen, d.h. Formen durch Ziehen eines Rundmaterials durch eine entsprechende Matrize, hergestellt werden, wobei letzteres bevorzugt angewandt wird.

In Fig. 3 ist dagegen eine aus dem Stand der Technik bekannte Ausgestaltung eines Kraftübertragungselementes 30' dargestellt, das in einem Rohrschaft 20' angeordnet ist. Dieses herkömmliche Kraftübertragungselement 30' ist wie der Innenraum des Rohrschafts 20' im Querschnitt rund ausgebildet, wobei der Durchmesser des Kraftübertragungselementes 30' im wesentlichen gleich dem Innendurchmesser des Rohrschaftes 20' ist. Dementsprechend ist zwischen dem Kraftübertragungselement 30' und dem Rohrschaft 20' kein Raum für den Durchtritt einer Spülflüssigkeit vorhanden, d.h. der Innenraum des Rohrschaftes 20' kann nicht gespült werden. Würde das Kraftübertragungselement 30' an zwei gegenüberliegenden Umfangsseiten bis auf die in Fig. 3 dargestellten unterbrochenen Linien beidseitig zu einem Band abgeflacht werden, würde die Anordnung aus dem Kraftübertragungselement 30' und dem Rohrschaft 20' einen erheblichen Stabilitätsverlust erleiden. Beim Betätigen des Kraftübertragungselementes 30' würde eine derartig beidseitig abgeflachte Ausgestaltung bewirken, daß das Kraftübertragungselement 30', insbesondere bei der Übertragung hoher Zugkräfte, in dem Rohrschaft 20' hin- und herspringt, wodurch sich der Rohrschaft 20' verbiegen kann. Bei einer zweiseitig abgeflachten Ausgestaltung des Kraftübertragungselementes 30' wäre zwar demnach ein Spülraum vorhanden, jedoch könnte ein solches Rohrschaftinstrument den mitunter hohen Kraftbeanspruchungen auf Dauer nicht standhalten.

Demgegenüber wird durch die in Fig. 2 dargestellte erfindungsgemäße Ausgestaltung des Kraftübertragungselementes 30 einerseits ein ausreichender Spülquerschnitt zwischen dem Kraftübertragungselement 30 und dem Rohrschaft 20 geschaffen, andererseits verhindern die radial bis an die Innenwand 44 des Rohrschaftes 20 reichenden Umfangsstellen 38, 40 und 42, daß das Kraftübertragungselement 30 bei den abwechselnden Zug- und Druckbeanspruchungen hin- und herspringt und bewirken somit eine Stabilisierung des Rohrschaftes 20, so daß dieser auch bei hohen Kraftbeanspruchungen nicht verbiegt.

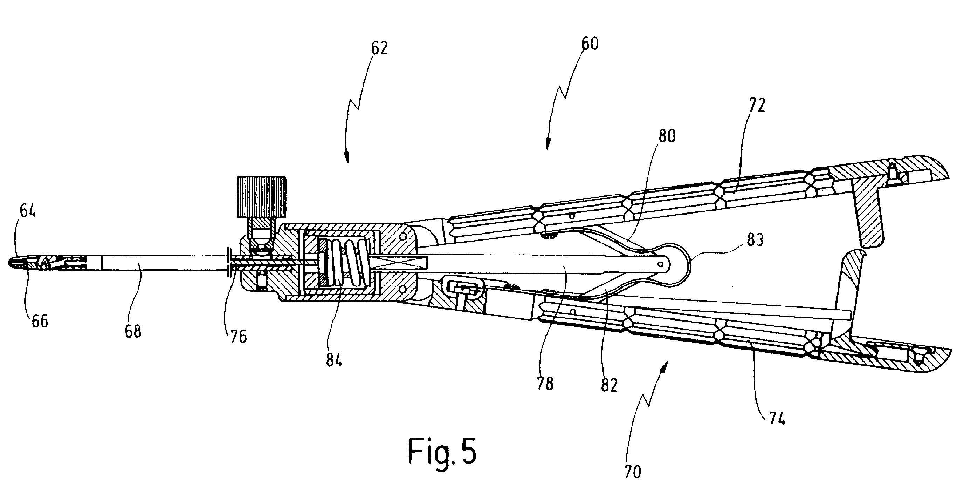

In Fig. 5 ist gemäß einem weiteren Ausführungsbeispiel ein medizinisches Rohrschaftinstrument 60 in Form eines Nadelhalters 62 dargestellt, bei dem die Erfindung ebenfalls vorteilhaft angewendet ist.

Mit dem Nadelhalter 62 wird zum Verbinden von Gewebe mittels einer Naht eine Nadel gehalten und durch das Gewebe geführt. Dazu weist der Nadelhalter 62 an seinem distalen Ende zwei bewegliche Werkzeuge 64 und 66 in Form zweier zangenartiger Maulteile auf, mit denen eine nicht dargestellte Nadel sicher und fest gehalten und geführt werden kann. Die beiden beweglichen Werkzeuge 64 und 66 sind am distalen Ende eines Rohrschaftes 68 angeordnet, an dessen proximalem Ende wiederum eine Handhabe 70 angeordnet ist, die in diesem Fall zwei bewegliche Griffteile 72 und 74 aufweist.

Die beweglichen Werkzeuge 64 und 66 sind über ein Kraftübertragungselement 76, das sich durch den Rohrschaft 68 hindurch erstreckt, und über ein mit dem Kraftübertragungselement 76 verbundenes Hebelgestänge, das sich aus einer axialen Stange 78, und zwei Schwenkhebeln 80 und 82 zusammensetzt, kraftschlüssig mit den beweglichen Griffteilen 72 und 74 verbunden. Die axiale Stange 78 ist durch eine an den Griffteilen 72 und 74 angreifende, nahe der Schwenkhebel 80 und 82 angebrachte Blattfeder 83 in ihre maximal distale Position vorgespannt. Eine Druckfeder 84 dient als Überlastschutz, der wirksam wird, wenn auf die Griffteile 72 und 74 eine zu große Kraft ausgeübt wird.

Durch Zusammendrücken der Griffteile 72 und 74 wird die axiale Stange 78 gegen die Wirkung der Blattfeder 83 nach proximal verschoben, wodurch auch das mit der axialen Stange 78 verbundene Kraftübertragungselement 76 zum Schließen der Werkzeuge 64 und 66 nach proximal verschoben wird.

Das Kraftübertragungselement 76 weist nun erfindungsgemäß zumindest in seinem sich durch den Rohrschaft 68 erstreckenden Bereich eine Ausgestaltung auf, wie sie aus Fig. 2 für das zuvor beschriebene Ausführungsbeispiel des Rohrschaftinstrumentes 10 vorgesehen ist. Somit besteht auch für den Nadelhalter 62 eine verbesserte Möglichkeit einer Reinigung, da zwischen dem Kraftübertragungselement 76 und dem Rohrschaft 68 einerseits eine Spülflüssigkeit zum Spülen des Innenraumes des Rohrschaftes 68 durchtreten kann. Andererseits wird die Stabilität des Rohrschaftes 68 dadurch jedoch nicht in Mitleidenschaft gezogen, d.h. dieser ist gegen ein Verbiegen geschützt, wie dies bereits an dem vorherigen Ausführungsbeispiel erläutert wurde.

## Patentansprüche

1. Medizinisches Rohrschaftinstrument, mit einem langerstreckten Rohrschaft (20; 68), mit zumindest einem beweglichen Werkzeug (16; 64, 66) am distalen Ende, weiterhin mit zumindest einem beweglichen Griffteil (24; 72, 74) am proximalen Ende, und schließlich mit einem langerstreckten Kraftübertragungselement (30; 76), dessen distales Ende (32) mit dem zumindest einen Werkzeug (16; 64, 66) und dessen proximales Ende (34) mit dem zumindest einen beweglichen Griffteil (24; 72, 74) kraftschlüssig verbunden ist, wobei sich das Kraftübertragungselement (30; 76) durch den Rohrschaft (20; 68) erstreckt, **dadurch gekennzeichnet, daß** das Kraftübertragungselement (30; 76) zumindest in seinem sich durch den Rohrschaft (20; 68) erstrekkenden Bereich zumindest abschnittsweise an zumindest drei Umfangsstellen (38, 40, 42) radial bis an eine Innenwand (44) des Rohrschaftes (20; 68) reicht und zwischen den drei Umfangsstellen (38, 40, 42) von der Innenwand (44) einen lichten Abstand aufweist, und in seinen übrigen sich durch den Rohrschaft (20; 68) erstreckenden Abschnitten zumindest teilumfänglich einen lichten Abstand von der Innenwand (44) aufweist.

2. Rohrschaftinstrument nach Anspruch 1, **dadurch gekennzeichnet, daß** sich die zumindest drei radial bis an die Innenwand (44) des Rohrschaft (20; 68) reichenden Umfangsstellen (38, 40, 42) durchgehend über den gesamten, sich durch den Rohrschaft (20; 68) erstreckenden Bereich des Kraftübertragungselementes (30; 76) erstrecken.

3. Rohrschaftinstrument nach Anspruch 1 oder 2, **dadurch gekennzeichnet, daß** das Kraftübertragungselement (30; 76) an den zumindest drei Umfangsstellen (38, 40, 42) abgerundet ist.

4. Rohrschaftinstrument nach Anspruch 3, **dadurch gekennzeichnet, daß** der Krümmungsradius der Umfangsstellen (38, 40, 42) kleiner ist als der Innenradius des Rohrschaftes (20; 68).

5. Rohrschaftinstrument nach einem der Ansprüche 1 bis 4, **dadurch gekennzeichnet, daß** das Kraftübertragungselement (30; 76) zwischen den zumindest drei Umfangsstellen (38, 40, 42) zur Längsmittelachse des Kraftübertragungselements (30; 76) hin gesehen konkav vertieft ist.

6. Rohrschaftinstrument nach einem der Ansprüche 1 bis 5, **dadurch gekennzeichnet, daß** sich die zumindest drei radial bis an die Innenwand (44) des Rohrschaftes (20; 68) reichenden Umfangsstellen (38, 40, 42) geradlinig erstrecken.

7. Rohrschaftinstrument nach einem der Ansprüche 1 bis 6, **dadurch gekennzeichnet, daß** die zumindest drei Umfangsstellen (38, 40, 42) einen gleichen Winkelabstand zueinander einnehmen.

8. Rohrschaftinstrument nach einem der Ansprüche 1 bis 7, **dadurch gekennzeichnet, daß** es eine Zange (12) zum Schneiden und/oder Fassen ist.

9. Rohrschaftinstrument nach einem der Ansprüche 1 bis 7, **dadurch gekennzeichnet, daß** es ein Nadelhalter (62) ist.

## Claims

1. A medical tubular-shaft instrument, comprising an elongated tubular shaft (20; 68), at least one movable tool (16; 64, 66) at the distal end, further at least one movable grip element (24; 72, 74) at the proximal end, and lastly an elongated force transmission element (30; 76), the distal end (32) of which is connected to the at least one tool (16; 64, 66) in force-locking fashion and the proximal end (34) of which is connected to the at least one movable grip element (24; 72, 74) in force-locking fashion, the force transmission element (30; 76) extending through the tubular shaft (20; 68), **characterized in that** the force transmission element (30; 76), at least in its region extending through the tubular shaft (20; 68) and at least locally, reaches radially at at least three circumferential locations (38, 40, 42) as far as an inner wall (44) of the tubular shaft (20; 68) and between the three circumferential locations (38, 40, 42) has a clearance from the inner wall (44), and in its remaining portions extending through the tubular shaft (20; 68) has a clearance from the inner wall (44) at least over part of its circumference.

2. The tubular-shaft instrument of Claim 1, **characterized in that** the at least three circumferential locations (38, 40, 42) reaching radially as far as the inner wall (44) of the tubular shaft (20; 68) extend continuously over the entire region of the force transmission element (30; 76) extending through the tubular shaft (20; 68).

3. The tubular-shaft instrument of Claim 1 or 2, **characterized in that** the force transmission element (30; 76) is rounded at the at least three circumferential locations (38, 40, 42).

4. The tubular-shaft instrument of Claim 3, **characterized in that** the radius of curvature of the circumferential lociations (38, 40, 42) is smaller than the inside radius of the tubular shaft (20; 68).

5. The tubular-shaft instrument of anyone of Claims 1 through 4, **characterized in that** the force transmission element (30; 76) is concavely recessed, viewed toward the longitudinal center axis of the force transmission element (30; 76), between the at least three circumferential locations (38, 40, 42).

6. The tubular-shaft instrument of anyone of Claims 1 through 5, **characterized in that** the at least three circumferential locations (38, 40, 42) reaching radially as far as the inner wall (44) of the tubular shaft (20; 68) extend in straight-line fashion.

7. The tubular-shaft instrument of anyone of Claims 1 through 6, **characterized in that** the at least three circumferential locations (38, 40, 42) assume an identical angular spacing from one another.

8. The tubular-shaft instrument of anyone of Claims 1 through 7, **characterized in that** it is a forceps (12) for cutting and/or grasping.

9. The tubular-shaft instrument of anyone of Claims 1 through 7, **characterized in that** it is a needle holder (62).

## Revendications

1. Instrument médical à tige tubulaire, comportant une tige tubulaire (20 ; 68) allongée, au moins un outil mobile (16; 64, 66) sur l'extrémité distale, en outre au moins un élément de préhension (24 ; 72, 74) mobile sur l'extrémité proximale, et enfin un élément de transmission de force (30 ; 76) allongé, dont l'extrémité distale (32) est assemblée par coopération de force avec ledit au moins un outil (16 ; 64, 66) et dont l'extrémité proximale (34) est assemblée par coopération de force avec ledit au moins un élément de préhension (24 ; 72, 74) mobile, l'élément de transmission de force (30 ; 76) s'étendant à travers la tige tubulaire (20 ; 68), **caractérisé en ce que** l'élément de transmission de force (30 ; 76), au moins dans sa partie passant à travers la tige tubulaire (20 ; 68), s'étend dans le sens radial au moins par zones avec au moins trois emplacements périphériques (38, 40, 42), jusqu'à une paroi intérieure (44) de la tige tubulaire (20 ; 68) et, entre les trois emplacements périphériques (38, 40, 42), est situé à distance de la paroi intérieure (44) et, dans ses autres parties qui passent à travers la tige tubulaire (20 ; 68), est situé au moins sur une partie du pourtour à distance de la paroi intérieure (44).

2. Instrument à tige tubulaire selon la revendication 1, **caractérisé en ce que** lesdits au moins trois emplacements périphériques (38, 40, 42), qui s'étendent dans le sens radial jusqu'à la paroi intérieure (44) de la tige tubulaire (20 ; 68), s'étendent en continu sur toute la partie de l'élément de transmission de force (30 ; 76) qui passe à travers la tige tubulaire (20 ; 68).

3. Instrument à tige tubulaire selon la revendication 1 ou 2, **caractérisé en ce que** l'élément de transmission de force (30 ; 76) est arrondi sur lesdits au moins trois emplacements périphériques (38, 40, 42).

4. Instrument à tige tubulaire selon la revendication 3, **caractérisé en ce que** le rayon de courbure des emplacements périphériques (38, 40, 42) est inférieur au rayon intérieur de la tige tubulaire (20 ; 68).

5. Instrument à tige tubulaire selon l'une quelconque des revendications 1 à 4, **caractérisé en ce que** l'élément de transmission de force (30 ; 76), entre lesdits au moins trois emplacements périphériques (38, 40, 42), est creusé selon une forme concave par référence à l'axe médian longitudinal de l'élément de transmission de force (30 ; 76).

6. Instrument à tige tubulaire selon l'une quelconque des revendications 1 à 5, **caractérisé en ce que** lesdits au moins trois emplacements périphériques (38, 40, 42), qui s'étendent dans le sens radial jusqu'à la paroi intérieure (44) de la tige tubulaire (20 ; 68), sont droits.

7. Instrument à tige tubulaire selon l'une quelconque des revendications 1 à 6, **caractérisé en ce que** lesdits au moins trois emplacements périphériques (38, 40, 42) incluent entre eux une même distance angulaire.

8. Instrument à tige tubulaire selon l'une quelconque des revendications 1 à 7, **caractérisé en ce qu'**il s'agit d'une pince (12) pour couper et/ou saisir.

9. Instrument à tige tubulaire selon l'une quelconque des revendications 1 à 7, **caractérisé en ce qu'**il s'agit d'un porte-aiguille (62).
